(19) Europäisches Patentamt
European Patent Office
Office européen des brevets

(11) EP 3 733 646 A1

(12) EUROPEAN PATENT APPLICATION
published in accordance with Art. 153(4) EPC

(43) Date of publication:
04.11.2020 Bulletin 2020/45

(51) Int Cl.:
C07C 319/28 $^{(2006.01)}$    C07C 323/58 $^{(2006.01)}$

(21) Application number: 18897708.6

(22) Date of filing: 25.12.2018

(86) International application number:
PCT/JP2018/047470

(87) International publication number:
WO 2019/131590 (04.07.2019 Gazette 2019/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 27.12.2017 JP 2017251240

(71) Applicant: SUMITOMO CHEMICAL COMPANY,
LIMITED
Chuo-ku
Tokyo
104-8260 (JP)

(72) Inventors:
• YAMASHIRO, Naoya
Niihama-shi
Ehime 7928521 (JP)
• MORIMOTO, Akira
Niihama-shi
Ehime 7928521 (JP)
• SATOH, Yoshitaka
Niihama-shi
Ehime 7928521 (JP)

(74) Representative: J A Kemp LLP
14 South Square
Gray's Inn
London WC1R 5JJ (GB)

(54) REFINED METHIONINE PRODUCTION METHOD

(57) An object of the present invention is to provide a refined methionine production method capable of achieving an improvement in washing efficiency. This production method is a method for producing refined methionine from a slurry of crude methionine and comprises (1) a step of separating the slurry of crude methionine into solid and liquid by using a continuous filter, (2) a step of spraying preliminary washing water on a crude cake obtained by separating the slurry of crude methionine into solid and liquid to wash the crude cake, and (3) a step of mixing a semi-refined cake obtained by spraying preliminary washing water on the crude cake to wash the crude cake with finishing washing water to wash the semi-refined cake.

**Description**

TECHNICAL FIELD

This patent application claims priority under the Paris Convention based on Japanese Patent Application No. 2017-251240 (filed on December 27, 2017) incorporated herein by reference in its entirety.
The present invention relates to a refined methionine production method.

BACKGROUND ART

There is disclosed, for example the following methionine production method, i.e, a method comprising hydrolyzing 5-(2-methylmercaptoethyl)hydantoin (hereinafter also referred to as methionine hydantoin) to obtain a liquid containing a methionine salt (hereinafter also referred to as a hydrolysis reaction liquid), and introducing carbon dioxide into the hydrolysis reaction liquid to precipitate methionine and thereby obtain a methionine slurry (hereinafter also simply referred to as a slurry).
In this production method, powdered methionine is obtained as a product by washing the methionine cake resulting from solid-liquid separation of the slurry, and then drying.
The methionine cake (hereinafter also referred to as a crude cake) obtained by solid-liquid separation of the slurry described above contains impurities such as potassium. Therefore, to obtain high-quality methionine having a low content percentage of the impurities (hereinafter also referred to as refined methionine), various studies have been conducted on a method for washing a crude cake (e.g., patent document 1).
In Patent Document 1 disclosed is washing of the crude cake resulting from solid-liquid separation of the slurry using a batch centrifugal filter, in which the washing was carried out by spraying washing water on it in the centrifugal filter

PRIOR ART DOCUMENT

PATENT DOCUMENT

Patent Document 1: Japanese Laid-Open Patent Publication No. 2013-173717

SUMMARY OF THE INVENTION

PROBLEM TO BE SOLVED BY THE INVENTION

The present inventors have conducted a study on replacing a batch filter used for solid-liquid separation of a slurry with a continuous filter so as to improve productivity of methionine. In this study, when the slurry is subjected to solid-liquid separation using a continuous filter to obtain a crude cake, and the crude cake is washed by a spray in the filter, it has been found that removal of impurities contained in the crude cake is difficult and that a large amount of washing water is required to attain the removal at a desirable level.
The present invention has been conceived in view of the situations, and an object thereof is to provide a refined methionine production method capable of achieving an improvement in impurity removal rate per unit amount of washing water, i.e., an improvement in methionine washing efficiency, when a continuous filter is used.

MEANS FOR SOLVING PROBLEM

As a result of intensive studies on the problem with removal of impurities contained in a crude cake in case of using a continuous filter, it has been found that the impurities in the crude cake include impurities incorporated in methionine crystal grains and other impurities, and the present inventors have found that although the latter impurities can easily be removed by washing with a spray, it was hard to remove the former impurities and that the methionine crystal grains must be kept in contact with washing water for a certain period of time to remove these impurities, thereby completing the present invention. Therefore, the refined methionine production method according to the present invention is a method for producing refined methionine from a slurry of crude methionine, comprising

(1) a step of separating the slurry of crude methionine into solid and liquid by using a continuous filter,
(2) a step of spraying preliminary washing water on a crude cake resulting from the solid liquid separation of the slurry of crude methionine, thereby washing the crude cake, and
(3) a step of mixing a semi-refined cake resulting from the washing by spraying preliminary washing water on the crude cake, with finishing washing water, thereby washing the semi-refined cake.

This production method is characterized in that the crude cake obtained by separating the slurry into solid and liquid by using a continuous filter is washed by spraying with preliminary washing water and is then washed by mixing with the finishing washing water. In this production method, impurities present on surfaces of crystal grains of methionine are removed by washing, comprising spraying the preliminary washing water on the crude cake, and impurities present in the crystal grains of methionine are removed by washing, comprising mixing the semi-refined cake with the finishing washing water. In this production method, the method for washing comprising spraying washing water on the cake (hereinafter also referred to as spray washing) the method for washing comprising mixing the cake and washing water (hereinafter also referred to as reslurry washing) are performed in combination, and the impurities contained in the crude cake can be removed with a reduced amount of washing water as compared to a method for washing with only one of the spray washing and the reslurry washing. This production method can achieve an improvement in methionine washing efficiency when a continuous filter is used.

EFFECT OF THE INVENTION

As is clear from the above description, the production method of the present invention can achieve an improvement in the methionine washing efficiency when a continuous filter is used. This production method can provide high-quality refined methionine having a less impurity content percentage while reducing an amount of washing water even when the continuous filter is used.

MODE FOR CARRYING OUT THE INVENTION

The present invention will now be described in detail based on a preferred embodiment. In this description, conventionally known portions will not be described in detail except those necessary for describing the present invention.

[Refined Methionine Production Method]

A production method of the present invention is a method for producing refined methionine from a slurry of crude methionine, comprising a separation step, a preliminary washing water spraying step, and a finishing washing water mixing step.

Examples of the method for producing a slurry of crude methionine include, for example a method in which a crystallization step is performed after the hydrolysis step described below.

[Hydrolysis Step]

In the hydrolysis step, methionine hydantoin is hydrolyzed in the presence of an alkali compound such as potassium hydroxide, sodium hydroxide, potassium carbonate, and potassium hydrogencarbonate. As a result, a liquid containing a methionine salt (hereinafter also referred to as a hydrolysis reaction liquid) is obtained. In this hydrolysis step, the pressure is set in a range of about 0.5 to 1.0 MPaG. The temperature is set in a range of 150 to 200 °C. Examples of the method for producing the raw material methionine hydantoin include a method in which 3-methylmercaptopropionaldehyde cyanohydrin is reacted with carbon dioxide and ammonia in water.

[Crystallization Step]

In the crystallization step, carbon dioxide is introduced into the hydrolysis reaction liquid obtained in the hydrolysis step. This causes methionine to precipitate, resulting in a methionine slurry in which methionine crystal grains are dispersed. In the crystallization step, the crystallization temperature is usually 0 to 50 °C, preferably 10 to 30 °C. The crystallization time is basically a time until carbon dioxide is saturated in the reaction liquid so that methionine is sufficiently precipitated and is usually 30 minutes to 24 hours.

In the present invention, the methionine slurry obtained by introducing carbon dioxide into the hydrolysis reaction liquid is a slurry of crude methionine. The methionine concentration in the slurry of crude methionine is usually in a range of 5 to 30 mass% in terms of solid content concentration. The slurry of crude methionine contains an alkali compound such as potassium hydroxide used for hydrolysis, a dimer of methionine, and impurities such as glycine and alanine generated due to decomposition of methionine.

[Separation Step]

In the separation step, the slurry of crude methionine obtained in the crystallization step is subjected to solid-liquid separation to obtain a cake of crude methionine that is a solid component (i.e., a crude cake) and a mother liquor that is a liquid component. In this production method, a continuous filter (hereinafter also simply referred to as a filter) is used for solid-liquid separation of the slurry of crude methionine.

In the present invention, the filter is not particularly limited as long as the filter is a continuous filter as described below. Examples of the continuous filter include a pressure type filter etc.

In the separation step, the slurry of crude methionine is continuously supplied to the filter from a reaction tank in which the crystallization step is performed. In this filter, solid-liquid separation is continuously performed, and a plate-shaped crude cake is continuously formed. The thickness of this plate-shaped crude cake is set in a range of 15 to 100 mm. In this separation step, conditions for the solid-liquid separation are appropriately set such that the moisture content of the crude cake is approximately 40 mass% or less.

As described above, the slurry of crude methionine contains impurities. Therefore, the crude cake obtained by solid-liquid separation also contains impurities.

In the present invention, the crude cake contains impurities derived from the alkali compound such as potassium hydroxide, sodium hydroxide, potassium carbonate and potassium hydrogencarbonate used in the hydrolysis step. Therefore, the impurities derived from the alkali compound vary depending on a type of the alkali compound used. For example, when a basic potassium compound containing potassium carbonate, potassium hydrogencarbonate, and potassium hydroxide is used as the alkali compound, an impurity concentration is indicated by a potassium concentration. The impurity concentration of the crude cake in this case is usually within a range of 0.5 mass% to 8 mass%. This potassium concentration is represented by a ratio of the mass of potassium to the mass of the crude cake after drying. In the present invention, the mass of potassium is obtained by converting the amount of potassium ions measured by ion chromatography into the mass of potassium. Analysis conditions for measuring the amount of potassium ions are as follows.

(Ion chromatography analysis conditions)

Column: Dionex IonPac CS12A
Column size: 4 mm in inner diameter, 250 mm in length
Eluent: 18 mmol/L methanesulfonic acid

[Preliminary washing Water Spraying Step]

In the preliminary washing water spraying step, washing water is sprayed on the crude cake obtained in the separating step to wash the crude cake. In the present invention, the washing water used for washing the crude cake is preliminary washing water. In this production method, the preliminary washing water spraying step is performed in the filter used in the separation step described above or in an apparatus other than the filter. From the viewpoint of production efficiency, the preliminary washing water spraying step is preferably performed in the filter used in the separation step. In this production method, the preliminary washing water is sprayed on the continuously formed plate-shaped crude cake in the filter to wash the crude cake. As a result, the impurities contained in the crude cake are partially washed away. Therefore, the amount of impurities contained in the crude cake after washing is smaller than that before washing. In the present invention, the washed crude cake with the amount of impurities reduced by washing with the preliminary washing water is a semi-refined cake. In the preliminary washing water spraying step, the temperature of the preliminary washing water is usually set in a range of 5 to 35 °C as appropriate.

In this production method, a spray is used for spraying the preliminary washing water on the crude cake. In this production method, the spray for spraying the preliminary washing water on the crude cake is not particularly limited as long as a spray amount of the preliminary washing water can be adjusted so that the preliminary washing water can be sprayed all over the entire crude cake.

In this preliminary washing water spraying step, from the viewpoint of effective removal of impurities, the mass of the preliminary washing water sprayed on the crude cake is preferably 100 g or more, and preferably 300 g or less, relative to the mass of the crude cake after drying of 100 g. The mass of the preliminary washing water is more preferably 150 g or more, and more preferably 250 g or less, relative to the mass of the crude cake after drying of 100 g.

In this production method, water may be used as the preliminary washing water, or a methionine aqueous solution may be used as the preliminary washing water. From the viewpoint of preventing methionine constituting the crude cake from being dissolved into the preliminary washing water and of improving a yield of methionine in a product, the methionine aqueous solution is preferable as the preliminary washing water. In this case, the concentration of methionine in the preliminary washing water is preferably 1.0 mass% or more, more preferably 2.0 mass% or more. Since the saturated solubility of methionine in water at normal temperature and ordinary pressure is 3.0 mass%, the upper limit of the concentration of methionine in the preliminary washing water is 3.0 mass%. Therefore, the concentration of methionine in the preliminary washing water is 3.0 mass% or less. In the present invention, the concentration of methionine may be measured by liquid chromatography.

The water used in the preliminary washing water is not particularly limited. Examples of the water comprise distilled water, pure water, ion-exchanged water, condensed water of steam, and industrial water.

The moisture content of the semi-refined cake obtained by performing the preliminary washing water spraying step in a continuous filter is usually 20 mass% to 40 mass%. In refinement of methionine, a methionine washing efficiency can be improved by applying the production method of the present invention when the moisture content of the semi-refined cake falls within this range.

[Finishing washing Water Mixing Step]

In the finishing washing water mixing step, the semi-refined cake obtained at the preliminary washing water spraying step is further washed with washing water. In the present invention, the washing water used for washing the semi-refined cake is the finishing washing water. In the finishing washing water mixing step, the semi-refined cake discharged from the filter is put into a drum of a stirrer together with the finishing washing water. Subsequently, a stirring blade in this drum is rotated to stir and mix the semi-refined cake and the finishing washing water. By performing reslurry washing in this way, the crystal grains of methionine constituting the semi-refined cake and the finishing washing water can be brought into contact with each other for a certain amount of time so as to remove impurities remaining in the semi-refined cake, or specifically, impurities present in the crystal grains of methionine constituting the semi-refined cake. This provides a slurry of cleaned methionine, i.e., a slurry of refined methionine. In the finishing washing water mixing step, the finishing washing water may be put into the drum after the semi-refined cake is put in, or the semi-refined cake may be put into the drum after the finishing washing water is put in, or the semi-refined cake and the finishing washing water may be put in at the same time. The temperature of the finishing washing water is usually set in a range of 5 to 35 °C as appropriate.

In this production method, the stirrer is not particularly limited as long as the semi-refined cake and the finishing washing water can sufficiently be stirred and mixed in the stirrer.

In the finishing washing water mixing step, from the viewpoint of effective removal of impurities, the mass of the finishing washing water put into the drum together with the semi-refined cake is preferably 100 g or more, and preferably 300 g or less, relative to the mass of the semi-refined cake after drying of 100 g. The mass of the finishing washing water is more preferably 150 g or more, and more preferably 250 g or less, relative to the mass of the semi-refined cake after drying of 100 g.

In this production method, water may be used as the finishing washing water, or a methionine aqueous solution may be used as the finishing washing water. From the viewpoint of preventing methionine from being dissolved into the finishing washing water and of improving a yield of methionine in a product, the methionine aqueous solution is preferable as the finishing washing water.

In this case, the concentration of methionine in the finishing washing water is preferably 1.0 mass% or more, more preferably 2.0 mass% or more. Since the saturated solubility of methionine in water at normal temperature and ordinary pressure is 3.0 mass%, the upper limit of the concentration of methionine in the finishing washing water is 3.0 mass%. Therefore, the concentration of methionine in the finishing washing water is 3.0 mass% or less.

The water used in the finishing washing water is not particularly limited. Examples of the water comprise distilled water, pure water, ion-exchanged water, condensed water of steam, and industrial water.

In the finishing washing water mixing step, a time of stirring for mixing the semi-refined cake and the finishing washing water (hereinafter referred to as the stirring time) is appropriately determined in consideration of the influence on production costs and the effective removal of impurities. This stirring time is usually set in a range of 3 minutes to 30 minutes.

In this production method, the slurry of refined methionine obtained in the finishing washing water mixing step is usually subjected to solid-liquid separation using a conventionally known solid-liquid separator. As a result, a cake of refined methionine (hereinafter, also referred to as a refined cake) is obtained. Therefore, this production method further comprises a step of solid-liquid separation of the slurry of refined methionine (hereinafter also referred to as a second separation step). The methionine concentration in the slurry of refined methionine is usually about 20 to 45 mass% in terms of solid content concentration.

In this production method, the refined cake obtained in the second separation step is usually put into a conventionally known dryer. In this dryer, the refined cake is dried. This removes moisture and provides high-quality powder methionine, i.e., refined methionine, with reduced impurity content percentage. This production method further comprises a step of drying the refined cake obtained by solid-liquid separation of the slurry of refined methionine (hereinafter also referred to as a drying step).

In this production method, the method for drying the refined cake is not particularly limited as long as moisture is sufficiently removed so that the refined methionine is obtained in the drying step. In this production method, the drying step is configured to have the same contents as those of a drying step in a conventionally known production method. In this production method, the drying temperature is usually 50 to 150 °C, preferably 100 to 140 °C. The drying time is usually 10 minutes to 24 hours, preferably 30 minutes to 2 hours. The state in which moisture is sufficiently removed means a state in which the moisture content of the refined methionine is 5 mass% or less.

As described above, this production method is the method for producing refined methionine from a slurry of crude methionine and comprises the separation step, the preliminary washing water spraying step, the finishing washing water mixing step, the second separation step, and the drying step. Specifically, in this production method, the crude cake is continuously formed from the slurry of crude methionine in the separation step. In the preliminary washing water spraying step, this crude cake is washed. The semi-refined cake obtained by this washing is further washed by mixing with the finishing washing water in the finishing washing water mixing step, and the slurry of refined methionine is obtained. In the second separation step, the slurry of refined methionine is subjected to solid-liquid separation, and the refined cake is obtained. In the drying step, this refined cake is dried to obtain the refined methionine.

As is clear from the above description, the production method of the present invention can achieve an improvement in the methionine washing efficiency when a continuous filter is used. This production method can provide high-quality refined methionine having a low impurity content percentage while reducing an amount of washing water even when the continuous filter is used.

Examples

The present invention will hereinafter be described in more detail with examples etc.; however, the present invention is not limited only to these examples.

[Production of Slurry of Crude Methionine]

Methionine aldehyde and hydrocyanic acid were reacted at normal temperature under ordinary pressure to synthesize methionine cyanohydrin. Ammonium carbonate was reacted with this methionine cyanohydrin in water at 75 °C for 2.5 hours to obtain a hydantoin liquid containing 15 mass% methionine hydantoin and 3.6 mass% ammonia.

A nitrogen gas was blown into the hydantoin liquid as an inert gas.

A liquid (potassium concentration: about 7.5 mass%) obtained by mixing a basic potassium compound containing potassium carbonate, potassium hydrogencarbonate, and potassium hydroxide with the hydantoin liquid after the blowing of the nitrogen gas was continuously supplied from an upper portion of an autoclave (supply rate: 700 g/hour), and a hydrolysis reaction was performed while maintaining the pressure at 1.0 MPaG and the temperature at 180 °C to obtain a liquid containing a methionine salt (hereinafter referred to as a hydrolysis reaction liquid).

Carbon dioxide was introduced into the hydrolysis reaction liquid at 0.35 MPaG and 20 °C. As a result, methionine was precipitated to obtain a slurry of crude methionine.

[Example]

The slurry of crude methionine was subjected to solid-liquid separation to obtain a crude cake. The crude cake (40 g) was sprayed with preliminary washing water for washing the crude cake to obtain a semi-refined cake. The moisture content of this semi-refined cake was 27 mass%. A methionine aqueous solution (methionine concentration=2 mass%, potassium concentration=0.2 mass%) was used as the preliminary washing water. The amount of the preliminary washing water used was set to 1.8 times the mass of the crude cake after drying.

In this example, the semi-refined cake was mixed with finishing washing water to obtain a slurry. A methionine aqueous solution (methionine concentration=2 mass%) was used as the finishing washing water. The amount of the finishing washing water used was set to 2.2 times the mass of the semi-refined cake after drying. The stirring time was set to 3 minutes.

In this example, the slurry obtained by mixing the semi-refined cake with the finishing washing water was aerated and dehydrated to obtain a methionine cake. The cake was dried to obtain powdery methionine.

In this example, a solid-liquid separation step, a preliminary washing water spraying step, and a finishing washing water mixing step were performed. This is represented by "Y" in Table 1 below.

[Comparative Example 1]

The slurry of crude methionine was subjected to solid-liquid separation to obtain a crude cake. The crude cake (40 g) was mixed with preliminary washing water to obtain a slurry. A methionine aqueous solution (methionine concentration=2 mass%) was used as the preliminary washing water. The amount of the preliminary washing water used was set to 12.5 times the mass of the crude cake after drying. The stirring time was set to 3 minutes.

In Comparative Example 1, the slurry obtained by mixing the crude cake with the preliminary washing water was aerated and dehydrated to obtain a methionine cake. The cake was dried to obtain powdery methionine.

In Comparative Example 1, the solid-liquid separation step and a preliminary washing water mixing step were performed. This is represented by "Y" in Table 1 below. In Comparative Example 1, the preliminary washing water spraying step was not performed.

[Comparative Example 2]

The slurry of crude methionine was subjected to solid-liquid separation to obtain a crude cake. The crude cake (40 g) was sprayed with preliminary washing water for washing the crude cake to obtain a semi-refined cake. The moisture content of this semi-refined cake was 27 mass%. A methionine aqueous solution (methionine concentration=2 mass%, potassium concentration=0.2 mass%) was used as the preliminary washing water. The amount of the preliminary washing water used was set to 2.4 times the mass of the crude cake after drying.

In this Comparative Example 2, the semi-refined cake was further sprayed with finishing washing water to wash the semi-refined cake. A methionine aqueous solution (methionine concentration=2 mass%) was used as the finishing washing water. The amount of the finishing washing water used was set to 2.5 times the mass of the semi-refined cake after drying.

In Comparative Example 2, the semi-refined cake after washing was aerated and dehydrated to obtain a methionine cake.

The cake was dried to obtain powdery methionine.

In Comparative Example 2, the solid-liquid separation step, the preliminary washing water spraying step, and the finishing washing water spraying step were performed.

This is represented by "Y" in Table 1 below. In Comparative Example 2, the finishing washing water mixing step is not performed.

[Amount of Washing Water]

An amount of washing water, i.e., an amount of washing water used, was measured for each of Example and Comparative Examples 1 and 2. The result is shown in Table 1 below as a ratio of the amount of washing water used to the mass of the cake after drying. When this value is smaller, the amount of washing water used is smaller. When the preliminary washing water and the finishing washing water are used as the washing water, the amount of washing water used is represented by the total amount of both waters.

[Washing Efficiency]

The washing efficiency of each of Examples and Comparative Examples 1 and 2 is shown in Table 1 below. The washing efficiency was evaluated by dividing a potassium removal rate (hereinafter referred to as K removal rate) by the amount of washing water to calculate the K removal rate per unit amount of washing water. When this value is larger, the washing efficiency is higher.

The K removal rate was calculated by Eq. (1) below.

$$\text{Eq. (1)} \quad \text{K removal rate (\%)} = [(\text{potassium content percentage in methionine before washing} - \text{potassium content percentage in methionine after washing})/\text{potassium content percentage in methionine before washing}] \times 100$$

In Eq. (1), the potassium content percentage in methionine before washing is a value obtained by converting a potassium content percentage in the methionine cake after solid-liquid separation into a potassium content percentage in the dry methionine, and similarly, the potassium content percentage in methionine after washing is a value obtained by converting a potassium content percentage in the methionine cake after washing into a potassium content percentage in the dry methionine.

[Table 1]

|  | Comparative Example 1 | Comparative Example 2 | Example |
|---|---|---|---|
| solid-liquid separation | Y | Y | Y |
| preliminary washing water mixing | Y |  |  |

(continued)

|  | Comparative Example 1 | Comparative Example 2 | Example |
|---|---|---|---|
| preliminary washing water spraying |  | Y | Y |
| finishing washing water mixing |  |  | Y |
| finishing washing water spraying |  | Y |  |
| Amount of Washing Water [-] | 12.5 | 4.9 | 4.0 |
| K removal rate [%] | 96.1 | 91.4 | 96.3 |
| K removal rate [%] per unit amount of washing water | 7.7 | 18.7 | 24.1 |

As shown in Table 1, the washing efficiency is higher in Example than in Comparative examples 1 and 2. This evaluation result clearly shows that an improvement in methionine washing efficiency is achieved when a continuous filter is used.

INDUSTRIAL APPLICABILITY

The refined methionine production method described above can provide high-quality refined methionine having a low impurity content percentage while reducing an amount of washing water even when a continuous filter is used.

**Claims**

1. A method for producing refined methionine from a slurry of crude methionine, the method comprising:

   a step of separating the slurry of crude methionine into solid and liquid by using a continuous filter;
   a step of spraying preliminary washing water on a crude cake resulting from solid liquid separation of the slurry of crude methionine, thereby washing the crude cake; and
   a step of mixing a semi-refined cake resulting from the washing by spraying preliminary washing water on the crude cake, with finishing washing water, thereby washing the semi-refined cake.

2. The method according to claim 1, wherein the semi-refined cake has a moisture content of 20 mass% to 40 mass%.

## INTERNATIONAL SEARCH REPORT

| International application No. |
|---|
| PCT/JP2018/047470 |

**A. CLASSIFICATION OF SUBJECT MATTER**
Int.Cl. C07C319/28(2006.01)i, C07C323/58(2006.01)i

According to International Patent Classification (IPC) or to both national classification and IPC

**B. FIELDS SEARCHED**

Minimum documentation searched (classification system followed by classification symbols)
Int.Cl. C07C319/28, C07C323/58

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2015-526485 A (ZHEJIANG NHU COMPANY LTD.) 10 September 2015, claims, paragraph [0013], examples, fig. 1 & US 2015/0284323 A1, claims, paragraph [0012], embodiments, fig. 1 & WO 2014/032401 A1 & EP 2894147 A1 & CN 102796033 A & AU 2013307993 A & KR 10-2015-0084774 A | 1,2 |

☒ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 February 2019 (28.02.2019) | 19 March 2019 (19.03.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2018/047470

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2013-173717 A (SUMITOMO CHEMICAL CO., LTD.) 05 September 2013, claims, paragraph [0022], examples & US 2015/0038739 A1, claims, paragraph [0033], examples & WO 2013/129405 A1 & EP 2821396 A1 & CN 104136418 A | 1,2 |
| A | EP 3246310 A1 (SHANDONG NHUAMINO ACID CO., LTD. ET AL.) 22 November 2017, claims, examples & JP 2018-504453 A & US 2018/0043281 A1 & WO 2016/127707 A1 & CN 104744326 A & CA 2974447 A & KR 10-2017-0106400 A | 1,2 |
| A | 編集兼発行人 三澤忠則, 炉過 (上), 2001, pp. 10, 50, non-official translation (Editor and publisher MISAWA, Tadanori, "Filtration (first volume)") | 1,2 |
| A | 三浦 貢, 炉過装置, 1962, pp. 104, 105, non-official translation (MIURA, Mitsugi, "Filtration device") | 1,2 |
| A | 炉過－メカニズムと炉材・炉過助剤, 1992, pp. 343-345, non-official translation ("Filtration – Mechanism and filter media, and filter aid") | 1,2 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017251240 A
- JP 2013173717 A